# EUROPÄISCHE PATENTANMELDUNG

(11) **EP 0 544 643 A1**
(43) Veröffentlichungstag der Anmeldung: **02.06.1993**
(21) Anmeldenummer: 93100940.1
(22) Anmeldetag: 01.08.1989
(51) Int. Cl.: C12C 1/18, C12P 7/56, A23L 2/38

(54) **Fermentativ gewonnene Milchsäure, Verfahren zu ihrer Herstellung und ihre Verwendung**

(30) Priorität: 12.08.1988 DE 3827355
(62) Teilanmeldung aus: 89114172.3
(71) Anmelder: Döhler GmbH, D-64295 Darmstadt (DE)
(72) Erfinder: Bäuml, Josef, W-6100 Darmstadt-Arheilgen (DE)
(74) Vertreter: Werner, Hans-Karsten, Dr.Dipl.-Chem.

(57) **Zusammenfassung**

Fermentativ gewonnene Milchsäure ist erhältlich aus enzymatisch aufgeschlossener Malzmaische, die mit den Feststoffen einer Milchsäuregärung unterworfen, danach von den Feststoffen abgetrennt, sterilisiert und durch Eindampfen auf einen Gehalt von mindestens 3 Gew.-%, vorzugsweise mehr als 6 Gew.-%, Milchsäure konzentriert wird. Es ist möglich, durch Verwendung geeigneter Mikroorganismen den Gehalt an L+Milchsäure gegenüber racemischer Milchsäure zu erhöhen. Die Konzentrate können zur pH-Absenkung von Bieren, insbesondere von hellen Bieren, alkoholarmen und alkoholfreien Bieren, verwendet werden. Weiterhin sind sie zur Herstellung von angesäuerten Getränken, Backmitteln und Teigsäuerungsmitteln geeignet.

## Beschreibung

Gegenstand der vorliegenden Erfindung ist eine fermentativ gewonnene Milchsäure, die aus enzymatisch aufgeschlossener Malzmaische gewonnen wird und ihre Verwendung zur pH-Absenkung von Bieren, insbesondere von hellen Bieren, alkoholarmen und alkoholfreien Bieren, zur Herstellung von gesäuerten Getränken, Backmitteln und Teigsäuerungsmitteln.

Es ist bekannt, daß man durch Absenken des pH-Wertes im Brauprozeß die Haltbarkeit des Bieres erhöhen kann, stabileren Schaum und geschmackliche Verbesserungen erhält. Dies ist insbesondere wichtig bei hellen Bieren sowie alkoholarmen und alkoholfreien Bieren. Diese pH-Absenkung wurde bisher erzielt durch Einsatz von Sauermalz oder durch Verwendung von milchsaurer Würze, die im Nebenstromverfahren hergestellt und der Maische oder der Würze zugegeben wird.

Sauermalz wird im allgemeinen hergestellt, indem man eine biologisch gewonnene Milchsäurelösung mit Malz vermischt, fermentiert und durch Darren trocknet. Der Nachteil dieses Vorgehens besteht darin, daß kein gleichbleibender Säurewert erzielt wird. Weiterhin ist es notwendig, eine zusätzliche Malzsorte in der Brauerei zu lagern und zu dosieren. Da Sauermalz schon beim Einmaischen eingesetzt wird, liegt der niedrigere pH-Wert bereits während des Malzkörperaufschlusses und der Treberabtrennung vor. Um spürbare Absenkungen des pH-Wertes zu erreichen, ist es daher notwendig, relativ hohe Mengen, nämlich etwa 6 %, Sauermalz zuzugeben.

Bei der Verwendung von Milchsäurewürze im Nebenstromverfahren wird ein Teil der Würze mit Milchsäurebakterien fermentiert und nach Erreichen der gewünschten Säuremenge der Maische bzw. Würze zugesetzt. Nachteile dieses Verfahrens bestehen insbesondere darin, daß die Brauerei dem Sudhaus sterile ungehopfte Würze entnehmen muß, diese separat fermentieren und dann beim Maischen oder zu Beginn der Kochung wieder zusetzen muß. Dies bedeutet zusätzliche Arbeit, zusätzliche Investitionen und zusätzliche mikrobiologische Kontrolle der Kultur. Weiterhin ist es nicht möglich, diese milchsaure Würze erst nach der Würzekochung zuzudosieren, weil die bei der Milchsäuregärung im allgemeinen entstehenden unerwünschten Geschmacksstoffe wie Diacetyl durch das Würzekochen ausgedampft werden müssen, um einen Fehlgeschmack im Bier zu verhindern. Weiterhin ist es nötig, die Milchsäurebakterien durch das Würzekochen zu inaktivieren. Bei höheren Dosagen von Milchsäurewürze ist es nicht mehr möglich, den typischen Milchsäuregeschmack komplett auszudampfen. Schließlich erfordert dieses Verfahren das Kultivieren von potentiellen Bierschädlingen in der Brauerei und erhöht somit die Gefahr einer Bierinfektion durch Fehlbedienung der Anlagen. Da der Säurewert in milchsaurer Würze nicht sehr hoch ist, ist es notwendig, etwa 1 % Milchsäurewürze zuzugeben.

Es ist weiterhin bekannt, nicht nur alkoholhaltige, alkoholarme und alkoholfreie Biere sondern auch andere alkoholfreie Getränke enthaltend fermentativ gewonnene Milchsäure herzustellen. Dazu wird Brot, Malz und/oder Rohfrucht mit geeigneter Milchsäure erzeugenden Mikroorganismen fermentiert. Derartige Getränke sind beispielsweise bekannt aus der GB-OS 2,057,846, der DE-OS 30 46 773 und der EP-OS 0 199 105. Diese Getränke enthalten je nach Herstellungsverfahren und verwendeten Mikroorganismen ein Gemisch aus L(+)-Milchsäure und D(-)-Milchsäure, gegebenenfalls weitere fermentativ entstandene organische Säuren, Kohlenhydrate, Mineralstoffe und Geschmacksstoffe. Außer spezifischen Geschmacks- und Aromastoffen des jeweils verwendeten Ausgangsmaterials enthalten sie insbesondere auch mehr oder weniger große Mengen unerwünschter Gemschmacksstoffe, wie Acetoin und Diacetyl. Insbesondere diese Geschmacksstoffe und der ausgeprägt saure Geschmack durch die Milchsäure führen dazu, daß die bisher bekannten Getränke, enthaltend fermentativ gewonnene Milchsäure, von breiten Verbraucherkreisen nicht oder nur sehr zurückhaltend akzeptiert werden.

Schließlich ist bekannt, Backmittel und Teigsäuerungsmittel herzustellen, die mehr oder weniger große Mengen physiologisch unbedenkliche Säuren einschließlich Milchsäure enthalten. Diese Milchsäure ist entweder fermentativ in dem Backmittel oder Teigsäuerungsmittel entstanden, oder wird in Form reiner Säure zugesetzt.

Die Erfindung hat sich zunächst die Aufgabe gestellt, die pH-Absenkung im Brauprozeß mit Milchsäure sicherer, einfacher und kostengünstiger durchzuführen, wobei es von besonderem Vorteil ist, wenn dabei nicht gegen das Reinheitsgebot verstoßen wird.

Die Erfindung hat sich weiterhin die Aufgabe gestellt, in einfacher und zuverlässiger Weise eine fermentativ gewonnene Milchsäure zu gewinnen, die lebensmitteltechnologisch in breiter Form verwendet werden kann, insbesondere für die Herstellung von gesäuerten Getränken, Backmitteln und Teigsäuerungsmitteln.

Es wurde jetzt gefunden, daß diese Aufgaben überraschend einfach gelöst werden können durch Milchsäure, erhältlich aus enzymatisch aufgeschlossener Malzmaische, die mit den Feststoffen einer Milchsäuregärung unterworfen, danach von den Feststoffen abgetrennt, sterilisiert und durch Eindampfen auf einen Gehalt von mindestens 3 Gew.-%, vorzugsweise mehr als 6 Gew.-%, Milchsäure konzentriert wird.

Es hat sich gezeigt, daß es ohne weiteres möglich ist, auch Konzentrate herzustellen, die 10 bis 15 Gew.-% Milchsäure enthalten.

Es wurde weiterhin gefunden, daß es erfindungsgemäß möglich ist, Milchsäurekonzentrate herzustellen, in denen mindestens 60 %, vorzugsweise sogar mehr als 80 Gew.-%, der Milchsäure als L+Milchsäure vorliegen. Derartige Milchsäurekonzentrate sind nicht nur zur pH-Absenkung in der Brauerei geeignet, sondern darüber hinaus hervorragend einsetzbar zur Herstellung von gesäuerten Getränken, Backmitteln und Teigsäuerungsmitteln.

Das Verfahren zur Herstellung solcher Milchsäurekonzentrate erfolgt dadurch, daß
a) Malzschrot bei Temperaturen zwischen 55 und 80°C enzymatisch zu einer vergärbaren Maische aufgeschlossen
b) ohne Abtrennung der Feststoffe (Treber) bei 20 bis 50°C einer Milchsäuregärung unterworfen
c) von den Feststoffen abgetrennt
d) sterilisiert und
e) durch Eindampfen auf einen Gehalt von mindestens 3 Gew.-%, vorzugsweise mehr als 6 Gew.-%, Milchsäure konzentriert wird.

Von besonderer Bedeutung ist dabei, daß die Milchsäuregärung ohne Abtrennung der Feststoffe, der sogenannten Treber, erfolgt. Hierdurch erreicht man eine wesentlich höhere Ausbeute an Milchsäure pro eingesetzter Malzmenge, ohne dabei Qualitätseinbußen in Kauf nehmen zu müssen.

Besonders wirtschaftlich arbeitet das Verfahren, wenn der enzymatische Aufschluß des Malzschrotes in mehreren hintereinandergeschalteten Stufen mit steigenden Temperaturen erfolgt. Dies kann beispielsweise dadurch erfolgen, daß man die Maische nacheinander durch mehrere gerührte Gefäße leitet, die auf steigende Temperaturen zwischen 55 und 80°C erwärmt werden. Der enzymatische Aufschluß kann beispielsweise erfolgen durch die im Malz vorhandenen Enzyme. Es ist jedoch ohne weiteres möglich, auch diese Enzyme in angereicherter oder reiner Form dem Aufschlußverfahren zuzusetzen. In der Brauereitechnik nennt man diese Varianten Infusionsverfahren und Dekoktionsverfahren. Bei diesen Verfahren ist es auch möglich, einen gewissen Anteil des Malzes zu ersetzen durch Rohfrucht. So ist es im allgemeinen ohne weiteres möglich, 20 bis 30 Gew.-% des Malzes durch Rohfrucht zu ersetzen, ohne daß die Ausbeute an Milchsäure bezogen auf die eingesetzten Cerealien sinkt. Werden höhere Mengen Rohfrucht zugesetzt, sinkt entweder die Ausbeute an Milchsäure, oder es ist nötig, zum Aufschluß der Rohfrucht zusätzlich Enzyme in angereicherter oder reiner Form zuzusetzen. Selbstverständlich sind derartige Produkte nicht mehr zulässig für Biere, die nach dem Reinheitsgebot gebraut werden sollen. Für alle anderen lebensmitteltechnologischen Anwendungen der Milchsäurekonzentrate sind aber diese Maßnahmen zulässig und oftmals sogar wegen der erwünschten Variationen der Geschmacksnoten für die Getränke und Backwaren ausgesprochen erwünscht. Als Rohfrucht geeignete Cerealien sind außer Gerste Roggen und Weizen, vor allem Dinkel, Mais und Reis sowie Gemische derselben.

Es hat sich gezeigt, daß der enzymatische Aufschluß des Malzes (gegebenenfalls unter Zusatz von Rohfrucht) bei relativ hohen Konzentrationen von 10 bis 20 Gew.-% Malz in der Maische erfolgen kann, während die nachfolgende Milchsäuregärung bei deutlich geringeren Konzentrationen erfolgen sollte. Vorzugsweise wird daher die heiße Maische mit kaltem Wasser verdünnt und dabei gleichzeitig auf solche Temperaturen abgekühlt, die für die Milchsäuregärung geeignet sind. Vorzugswseise wird daher die Milchsäuregärung bei Konzentrationen von 3 bis 8 Gew.-% Malz in der Maische durchgeführt. 4 bis 5 Gew.-% Malz in der Maische liefern optimale Ergebnisss.

Die Milchsäuregärung erfolgt durch Animpfung mit einem Lactobacillus, wobei praktisch alle Iactobacillen in Frage kommen. Vorzugsweise werden solche eingesetzt, die überwiegend L+Milchsäure erzeugen. Da diese Lactobacillen jedoch meist schon bei höheren pH-Werten ihr Wachstum einstellen als Lactobacillen, die racemische Milchsäure erzeugen, kann es von besonderem Vorteil sein, zunächst mit einem Lactobacillus anzuimpfen, der überwiegend L+Milchsäure erzeugt, um dann in einer zweiten Stufe zusätzlich anzuimpfen mit einem Lactobacillus, der racemische Milchsäure erzeugt. Sofern Lactobacillen verwendet werden, die auch auf Malz vorkommen, kann das erfindungsgemäße Milchsäurekonzentrat ohne Verletzung des Reinheitsgebotes verwendet werden. Um zweistufig vorzugehen, kann dazu in der ersten Stufe mit einem auf Malz vorkommenden Lactobacillus casei und in einer zweiten Stufe mit einem auf Malz vorkommenden Lactobacillus buchneri angeimpft werden.

Die Milchsäuregärung wird bei Temperaturen zwischen 20 und 50°C durchgeführt, und zwar in Abhängigkeit von den verwendeten Lactobacillen. Vorzugsweise wird bei Temperaturen zwischen 30 und 40°C gearbeitet.

Im Anschluß an die Milchsäuregärung werden die Feststoffe abgetrennt, beispielsweise durch Abdekantieren, Klären, Filtrieren oder Zentrifugieren. Die so erhaltene wäßrige Phase enthält im allgemeinen 0,5 bis 1,5 Gew.-% Milchsäure sowie weitere wasserlösliche Bestandteile, insbesondere unvergorene Zucker, Oligosaccharide, Aminosäuren, wasserlösliche Eiweißstoffe und in Wasser lösliche Salze. Weiterhin enthält sie oftmals noch unerwünschte und geschmacklich störende Nebenprodukte der Milchsäuregärung. Diese wäßrige Phase wird erfindungsgemäß sterilisiert und durch Eindampfen konzentriert und dabei gleichzeitig von den unerwünschten Geschmacksstoffen befreit.

Das Sterilisieren kann beispielsweise durch Sterilfiltration in einem Plattenpasteur erfolgen. Anschließend wird durch Eindampfen, vorzugsweise bei vermindertem Druck, aufkonzentriert auf mindestens 3 Gew.-%, vorzugsweise mehr als 6 Gew.-%, Milchsäure. Es ist ohne weiteres möglich, auch Konzentrate mit 10 bis 15 Gew.-% Milchsäure herzustellen, wobei dann auf einen Feststoffgehalt von 40 bis 70 % konzentriert wird. Schließlich ist es möglich, die Konzentrate zur völligen Trockne einzudampfen und dann in fester Form zu lagern, zu transportieren und später wieder einzusetzen. Insbesondere für den Sektor Brauereien, Getränke, Backmittel und Teigsäuerungsmittel bieten sich aber Konzentrate mit einem Feststoffgehalt zwischen 30 und 65 Gew.-% an. Derartige Konzentrate sind rasch und problemlos wieder in Wasser oder Bier auflösbar. Sie können problemlos gelagert, transportiert und zudosiert werden. Aufgrund ihres hohen Gehaltes an Milchsäure sind sie ausgezeichnet gegen Fremdinfektionen geschützt. Dennoch empfiehlt es sich, diese Konzentrate so abzufüllen und unter solchen Bedingungen wieder zuzudosieren, daß keine Fremdinfektionen in das Endprodukt Bier, gesäuertes Getränk oder Gebäck eingeschleppt werden.

Ein besonderer Vorteil der erfindungsgemäßen Milchsäurekonzentrate ist, daß sie bezüglich des Milchsäuregehaltes standardisiert werden können und deshalb automatisch zudosiert werden können. Es ist daher auch möglich, einen konstanten pH-Wert bei der Bierherstellung zu erreichen. Die Einsatzmengen sind im Vergleich zu Sauermalz und saurer Würze im Nebenstromverfahren sehr niedrig, es genügen nämlich meist schon Mengen um 0,05 bis 0,2 Gew.-%. Dies bedeutet auch, daß Transport und Lagerhaltung kostengünstig und unproblematisch sind. Auch bei längerer Lagerung innerhalb oder außerhalb der Brauerei ist kein Qualitätsverlust zu befürchten. Da die erfindungsgemäßen Milchsäurekonzentrate frei von lebenden oder inaktivierten Milchsäurebakterien sind, besteht keine Infektionsgefahr für die Bierherstellung. Das Milchsäurekonzentrat kann somit auch bei jedem beliebigen Verfahrensschritt der Bierherstellung, insbesondere nach der Würzekochung, zugefügt werden. Die unerwünschten Geschmacksstoffe der Milchsäuregärung sind beim Konzentrieren durch Eindampfung so weitgehend entfernt worden, daß sie nicht mehr geschmacklich stören. Es ist deshalb keine Beeinträchtigung des Geschmacksbildes des Bieres zu befürchten. Durch den Zusatz des Milchsäurekonzentrats nach dem Hopfenkochen beobachtet man eine bessere Hopfenisomerisation und kommt man mit niedrigerer Hopfendosage aus. Der bisher beobachtete Verlust an Milchsäure durch Ausschleusen mit dem Treber entfällt.

Insbesondere bei Spezialbieren, nämlich alkoholarmen und alkoholfreien Bieren sowie alkoholfreien Biermischgetränken, kann der Zusatz an Milchsäurekonzentrat deutlich erhöht werden. Damit kann der meist zu flache Geschmack, die unzureichende Schaumstabilität und die geringe Haltbarkeit vermieden werden. Insbesondere bei diesen Anwendungen ist das erfindungsgemäße Milchsäurekonzentrat den bisherigen Verfahren deutlich überlegen. Aufgrund des guten Geschmackes und der natürlichen Herkunft können die Milchsäurekonzentrate auch ausgezeichnet eingesetzt werden für gesäuerte Getränke, als Backmittel und Teigsäuerungsmittel, wobei Milchsäurekonzentrate mit mindestens 60 % L(+)-Milchsäure bevorzugt zum Einsatz kommen.

Schließlich ist es möglich, die Milchsäurekonzentrate mit Milch zu vermischen, wobei rasch joghurtartige Produkte bzw. Getränke entstehen. Ein weiterer Vorteil der Milchsäurekonzentrate ist der standardisierte Gehalt an sonstigen natürlichen Inhaltsstoffen der verwendeten Cerealien, nämlich Vitamine und Mineralien sowie der L(+)-Milchsäure.

Erfindungsgemäße Milchsäurekonzentrate und Verfahren zu ihrer Herstellung sind in den nachfolgenden Beispielen näher erläutert:

### Beispiel 1

25 kg gemahlenes oder geschrotetes Gerstenmalz werden mit 138 l entmineralisiertem Wasser angemaischt und 30 Min. bei 58°C gerührt. Diese Maische wird dann in eine zweite Temperaturstufe gepumpt, in der sie 30 Min. bei 62°C verbleibt. Diese Maische wird dann in eine dritte Temperaturstufe gepumpt, in der sie 30 Min. bei 72°C verbleibt. Schließlich wird sie in eine vierte Temperaturstufe gepumpt, in der sie 30 Min. bei 78°C verbleibt. Die so erhaltene enzymatisch aufgeschlossene Maische wird mit 278 l kaltem, entmineralisiertem Wasser vermischt, wobei eine verdünnte Maische von 4 bis 6° Brix und einer Temperatur von 30 bis 34°C erhalten wird. Diese Maische wird in einen ersten Fermentationstank mit einem Volumen von 20000 l gepumpt und mit einer Kultur von Lactobacillus casei ssp. casei eingeimpft. Geeignet sind beispielsweise die Stämme DSM 20321 und DSM 20011. In diesen Fermentationstank werden weitere Chargen Maische eingepumpt, wobei der Gesamtansatz durchschnittlich 2 Tage in diesem Tank verbleibt. Nach 2 Tagen Verweilzeit wird in in einen zweiten gleich großen Tank umgepumpt, in dem die Fermentation 2 Tage fortgesetzt wird. Dann wird in einen dritten Fermentationstank mit einem Volumen von ca. 35000 l umgepumpt und beispielsweise mit Lactobacillus buchneri DSM 20057 angeimpft. Nach ca. 3 Tagen wird ein optimaler Wert an Milchsäure erreicht. Die fermentierte Maische der dritten Stufe wird über einen Separator geklärt, in einem Platten-Pasteur pasteurisiert und in einem Vakuumverdampfer auf ca. 50 Gew.-% Feststoffgehalt konzentriert. Das erhaltene Konzentrat ist frei von unerwünschten Geschmacksstoffen und Alkoholen. Es enthält außer ca. 100 g Milchsäure (ca. 75 g L+Milchsäue) ca. 400 g weitere Feststoffe, insbesondere Restzucker, Oligosaccharide, Aminosäuren, Eiweiß und Salze. Das Milchsäurekonzentrat wird auf einen garantierten Sollwert eingestellt und anschließend heiß in Tanks, Fässer oder Dosen abgefüllt. Eine Zudosierung von ca. 0,1 % zu einem hellen Bier führt zur gleichen pH-Absenkung wie der Zusatz von 6 % Sauermalz.

### Beispiel 2

Das Verfahren gemäß Beispiel 1 wird wiederholt, jedoch bereits in der ersten Stufe mit einem Lactobacillus buchneri angeimpft. Man erhält ein Konzentrat, welches ca. 100 g racemische Milchsäure enthält, jedoch geschmacklich gleichwertig ist. Es kann daher in gleicher Weise zur pH-Absenkung von Bieren verwendet werden.

### Beispiel 3

Backversuche mit dem Konzentrat aus dem Beispiel 1 ergeben lebensmittelrechtlich und geschmacklich einwandfreie Backprodukte. Die Konzentrate können somit auch hierfür eingesetzt werden.

### Beispiel 4

Das Beispiel 1 wird wiederholt, jedoch wird anstelle von reinem Gerstenmalz ein Gemisch aus 80 Gew.-% Gerstenmalz aus biologischem Anbau, 10 Gew.-% Roggen und 10 Gew.-% Weizen auch jeweils aus biologischem Anbau eingestzt. Die aufgeschlossene Maische wird über einen Röhrenkühler auf 37°C abgekühlt und kontinuierlich in den ersten Fermenter eingelagert. Dort vermehrt sich die Kultur, so daß ein Ansetzen mit frischer Reinkultur nicht erforderlich ist. Der Fermenter wird nicht gerührt und reichert sich dadurch bei fortschreitender Befüllung mit Trebern an, bis diese nach Erreichen des Überlaufrohres in den zweiten und später in den weiteren Fermenter mitgerissen werden. Dadurch reichert sich der Gehalt an Treber an. Gleichzeitig steigt hierdurch die Ausbeute an Milchsäure, so daß nach dem Konzentrieren auf 65° Brix der Gehalt an Milchsäure von 100 bis 120 g bis auf einen Gehalt von 200 bis 260 g/kg Konzentrat ansteigt.

### Beispiel 5

Aus 60 g eines Milchsäurekonzentrats gemäß Beispiel 4 mit einem Gehalt von 150 g Milchsäure/kg Konzentrat, 200 g naturtrübem Apfelsaft und 740 g entmineralisiertem Wasser wird ein wohlschmeckendes Erfrischungsgetränk gemischt, welches reich an Kalium und Mineralstoffen, aber arm an Natriumionen ist, einen hohen Vitamingehalt, aber einen niedrigen Gehalt an Kohlenhydraten aufweist. Es wird pasteurisiert und heiß in Flaschen abgefüllt.

### Beispiel 6

Aus 60 g eines Milchsäurekonzentrats gemäß Beispiel 4 mit einem Gehalt von 150 g Milchsäure/kg Konzentrat, den nachfolgend aufgeführten handelsüblichen Grundstoffkonzentraten und entmineralisiertem Wasser zur Ergänzung auf jeweils 1 kg werden Erfrischungsgetränke hergestellt, die bei 75 bis 90°C heiß in Flaschen gefüllt werden. Es entstehen wohlschmeckende Erfrischungsgetränke. Als handelsübliche Grundstoffkonzentrate werden verwendet
15 g Orange/Maracuja (ca. 9:1) oder
35 g Multiexotic (Art. Nr. 5695 der Anmelderin) oder
125 g Vitasin (Handelsprodukt der Anmelderin aus Obstessig, Apfelsaft, Apfelsaftkonzentrat, Honig mit einem Gehalt von ca. 80 % Trockensubstanz) oder
85 g Apfel/Citrone (80:20).

Die Mischungen werden gewünschtenfalls mit Kohlensäure versetzt. Dazu werden sie erst in Flaschen gefüllt und dann in der Flasche bei 75 bis 90°C pasteurisiert. In diesem Fall kann zur Schonung der Inhaltsstoffe die Pasteurisationstemperatur auf 65 bis 70°C gesenkt werden.

Anstelle der oben genannten Obstsäfte, Fruchtsäfte oder Grundstoffkonzentrate können auch aus Gemüsesäften aus Karotten, Rote Beeten und Tomaten wohlschmeckende angesäuerte Getränke mit guter Haltbarkeit und ausgewogenem Verhältnis von Gechmacks- und Aromastoffen, Kohlenhydraten, Mineralstoffen und Vitaminen hergestellt werden, wobei ebenfalls die fertigen Gemische miteinander pasteurisiert werden.

Ein Vorteil aller mit den erfindungsgemäßen Milchsäurekonzentraten hergestellten Getränke besteht darin, daß sie nicht nur lebensmittelrechtlich sondern tatsächlich alkohlfrei hergestellt werden können, da auch Spuren von Alkohol, die bei der Milchsäuregärung entstehen, ausgedampft werden können. Dies kann für alkoholsüchtige Verbraucher von großer Bedeutung sein.

## Patentansprüche

1. Verfahren zur Herstellung eines Milchsäurekonzentrats, in dem Malzschrot zu Maische aufgeschlossen und diese einer Milchsäuregärung unterworfen wird, dadurch gekennzeichnet, daß
a) Malzschrot in mehreren hintereinandergeschalteten Stufen bei steigenden Temperaturen zwischen 55 und 80°C enzymatisch zu einer vergärbaren Maische mit einer Konzentration von 10 bis 20 Gew.-% Malz aufgeschlossen wird,
b) die Maische danach mit kaltem Wasser auf Konzentrationen von 3 bis 8 Gew.-% Malz verdünnt wird,
c) die Maische ohne Abtrennung der Feststoffe (Treber) bei 20 bis 50°C einer Milchsäuregärung unterworfen wird,
d) von den Feststoffen abgetrennt, sterilisiert und durch Eindampfen auf einen Gehalt von mindestens 3 Gew.-%, vorzugsweise mehr als 6 Gew.-%, Milchsäure konzentriert wird.

2. Verfahren gemäß Anspruch 1, dadurch gekennzeichnet, daß ein Teil des Malzes durch Rohfrucht ersetzt ist.

3. Verfahren gemäß Anspruche 1 bis 2, dadurch gekennzeichnet, daß die Fermentation unter Anreicherung der Treber erfolgt.

4. Verwendung des Milchsäurekonzentrats gemäß Ansprüchen 1 bis 3 zur pH-Absenkung von Bieren, insbesondere von hellen Bieren, alkoholarmen und alkoholfreien Bieren, zur Herstellung von gesäuerten Getränken, Backmitteln und Teigsäuerungsmitteln.

5. Verwendung gemäß Anspruch 4 zur Herstellung von gesäuerten Getränken mit einer Beimischung von Obstsaft, Fruchtsaft und/oder Gemüsesaft oder daraus hergestellten Grundstoffkonzentraten.
